# EUROPEAN PATENT APPLICATION

(11) **EP 1 666 479 A1**
(43) Date of publication of application: **07.06.2006**
(21) Application number: 04787849.1
(22) Date of filing: 10.09.2004
(51) Int. Cl.: C07D 413/06, A61K 31/422, A61P 3/04, A61P 3/06, A61P 3/10, A61P 9/00, A61P 9/10, A61P 9/12

(54) **CRYSTAL OF HETEROCYCLIC COMPOUND**

(30) Priority: 12.09.2003 JP 2003320556
(71) Applicant: Nippon Shinyaku Co., Ltd., Kyoto-shi, Kyoto 601-8550 (JP)
(72) Inventor: OKADAI, Takeshi, Kyoto-shi, Kyoto 6120882 (JP); NAKAMURA, Kazufumi, 6170826 (JP); HARABE, Tetsuji, 5200025 (JP)
(74) Representative: Vossius & Partner
(86) International application number: PCT/JP2004/013209
(87) International publication number: WO 2005/026160

(57) **Abstract**

The main purpose of the invention is to provide a new specific crystal of 2-methyl-c-5-[4-[5-methyl-2-(4-methylphenyl)-1,3-oxazol-4-yl]butyl]-1,3-dioxane-r-2-carboxylic acid.

The invention includes, for example, a crystal of 2-methyl-c-5-[4-[5-methyl-2-(4-methylphenyl)-1,3-oxazol-4-yl]butyl]-1,3-dioxane-r-2-carboxylic acid, which has peaks at the angles of diffraction of at least 12.4°, 17.1°, and 20.8° in a powder X-ray diffraction spectrum, and a preventive or therapeutic agent comprising the crystal as an active ingredient for use in coronary artery diseases, cerebral infarction, hyperlipemia, arteriosclerosis, diabetes mellitus, hypertension, or obesity.

## Description

### Technical Field

The present invention relates to crystals of 2-methyl-c-5-[4-[5-methyl-2-(4-methylphenyl)-1,3-oxazol-4-yl]butyl]-1,3-dioxane-r-2-carboxylic acid (hereinafter referred to as Compound A).

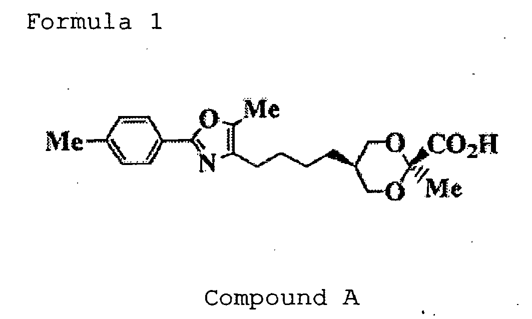

### Background Art

Compound A has blood triglyceride-lowering and low-density lipoprotein cholesterol (hereinafter referred to as LDL-C) lowering effects as well as blood sugar-lowering or blood insulin-lowering effect or high-density lipoprotein cholesterol (hereinafter referred to as HDL-C) increasing or arteriosclerosis index [a ratio of non-high-density lipoprotein cholesterol to HDL-C, calculated from (total cholesterol value - HDL-C value)/HDL-C] lowering effect. Thus Compound A is useful as a prophylactic or therapeutic agent for coronary artery diseases, cerebral infarction, hyperlipemia, arteriosclerosis, diabetes mellitus, hypertension, or obesity. These facts are described in Patent Document 1.

There is no description or suggestion, however, on the presence of polymorphism of the crystal in Patent Document 1, though the usefulness of Compound A as a therapeutic agent and the like for the above-mentioned diseases has been described.

In general, when polymorphism of crystal is present in a drug, difference of the crystalline form affords an influence on the efficacy, stability, and so on, of the drug; accordingly, in such a case, the drug should be provided in a possibly defined crystalline form.

Compound A can be prepared according to the following process (for example, see Patent Document 1).

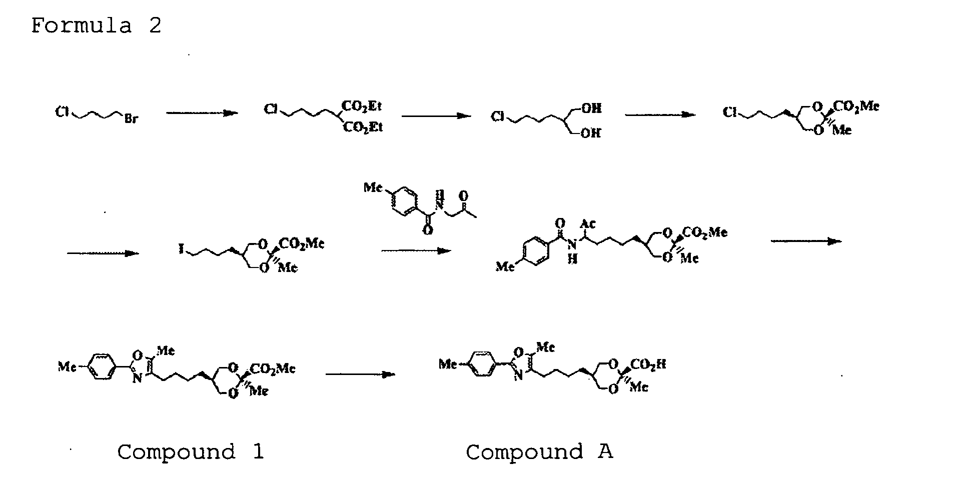

Compound A had been prepared in high purity from Compound 1, a synthetic intermediate, by purification by column chromatography and subsequent hydrolysis. In this process, however, the use of column chromatography in purification of a large quantity of synthetic product on an industrial scale necessarily requires use of a great deal of silica gel and a solvent or solvents, which greatly increases the production cost and gives an environmentally adverse effect caused by the solvents used. Thus, an alternative process for producing Compound A has strongly been desired, also in view of the complicated and troublesome operation of the current process.
[Patent Document 1]
International Patent Publication No. 01/90087 Pamphlet

### Disclosure of Invention

### Problem to be Solved by the Invention

The main purpose of the invention is to provide a new specific crystal of Compound A.

### Means for Solving the Problems

The present inventors have found as a result of their enthusiastic study that Compound A has plural crystalline forms and these crystals can be prepared individually. Thus the invention was completed.

The present invention includes the followings.
(1) A crystal of Compound A, which has peaks at the angles of diffraction of at least 4.9°, 13.7°, and 19.7° in a powder X-ray diffraction spectrum.
(2) The crystal as described in (1), further having peaks at the angles of diffraction of 15 5°, 18.6° and 21.0°.
(3) A crystal of Compound A, which has peaks at the angles of diffraction of at least 12.4°, 17.1°, and 20.8° in a powder X-ray diffraction spectrum.
(4) The crystal as described in (3), further having peaks at the angles of diffraction of 13,3°, 22.2° and 25.9°.
(5) A pharmaceutical composition comprising as an active ingredient the crystal as described in (1) or (2).
(6) A pharmaceutical composition comprising as an active ingredient the crystal as described in (3) or (4).
(7) A preventive or therapeutic agent comprising as an active ingredient the crystal as described in (1) or (2) for use in coronary artery diseases, cerebral infarction, hyperlipemia, arteriosclerosis, diabetes mellitus, hypertension, or obesity.
(8) A preventive or therapeutic agent comprising as an active ingredient the crystal as described in (3) or (4) for use in coronary artery diseases, cerebral infarction, hyperlipemia, arteriosclerosis, diabetes mellitus, hypertension, or obesity.
(9) A process for preparing the crystal as described in (3) or (4), which process comprises the steps of:
   (i) preparing the crystal as described in (1) or (2) from 2-methyl-c-5-[4-[5-methyl-2-(4-methylphenyl)-1,3-oxazol-4-yl]butyl]-1,3-dioxane-r-2-carboxylic acid; and
   (ii) preparing the crystal as described in (3) or (4) from the crystal obtained in the above step (i).

### Brief Description of Drawings

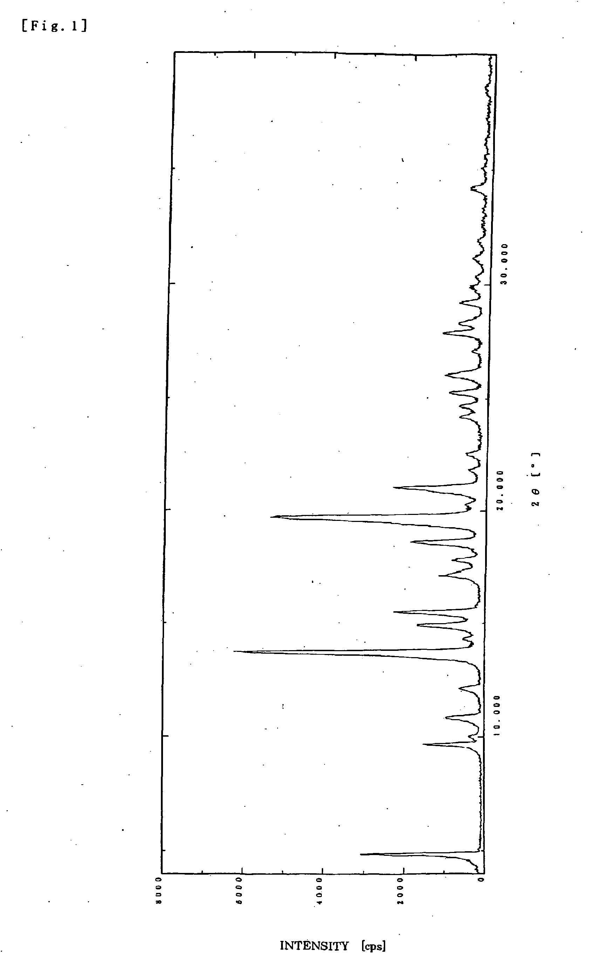

Fig. 1 shows a chart of powder X-ray diffraction spectrum in Form I crystal of Compound A. The vertical axis indicates the peak intensity (cps) and the horizontal axis indicates the angle of diffraction (2θ [°]) .

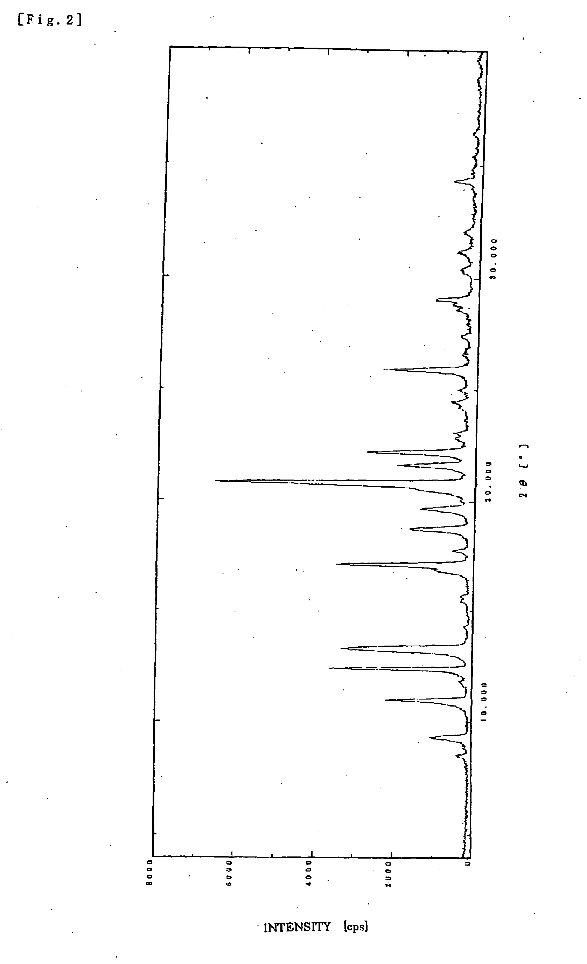

Fig. 2 shows a chart of powder X-ray diffraction spectrum in Form II crystal of Compound A. The vertical axis indicates the peak intensity (cps) and the horizontal axis indicates the angle of diffraction (2θ [°]).

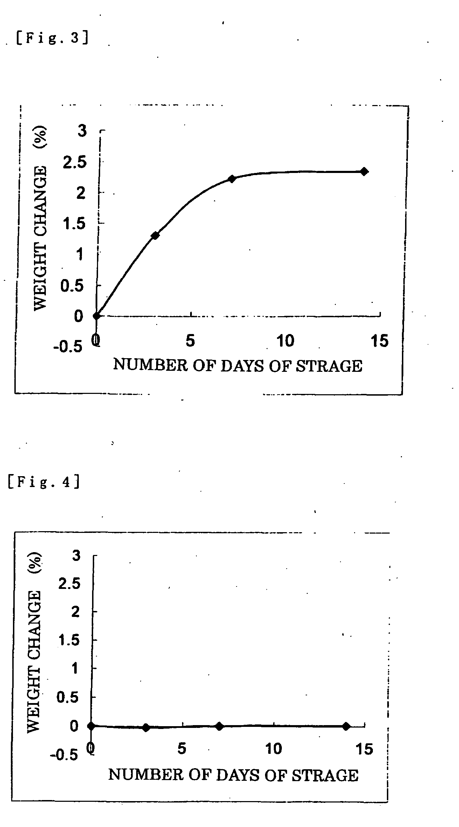

Fig. 3 shows a weight change of Form I crystal of Compound A with the elapse of time in a condition of 93% RH at 25°C. The vertical axis indicates the weight change (%) and the horizontal axis indicates the period of storage (the number of days) .
Fig. 4 shows a weight change of Form II crystal of Compound A with the elapse of time in a condition of 93% RH at 25°C. The vertical axis indicates the weight change (%) and the horizontal axis indicates the period of storage (the number of days) .

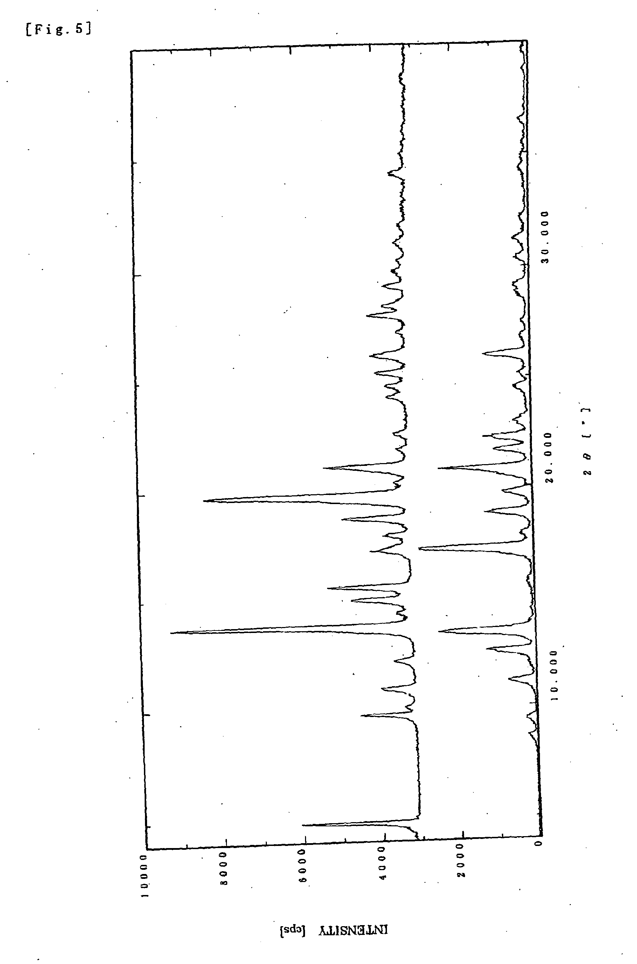

Fig. 5 shows the change in powder X-ray diffraction spectrum in Form I crystal after standing at 25°C in 93% RH for 14 days. The vertical axis indicates the peak intensity (cps) and the horizontal axis indicates the angle of diffraction (2θ [°]). The upper chart shows a powder X-ray diffraction spectrum before storage and the bottom one shows that after storage.

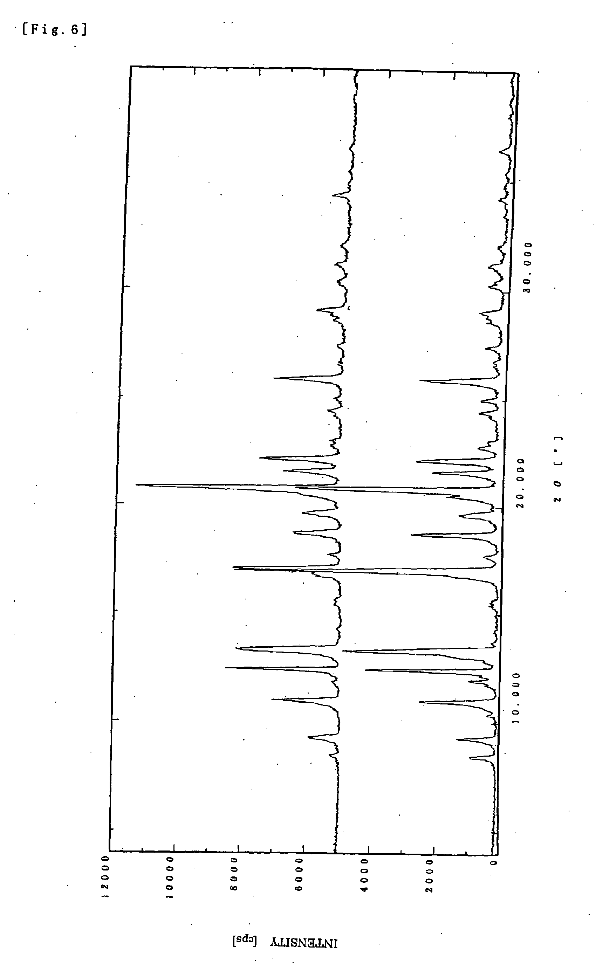

Fig. 6 shows the change in powder X-ray diffraction spectrum in Form II crystal after standing at 25°C in 93% RH for 14 days. The vertical axis indicates the peak intensity (cps) and the horizontal axis indicates the angle of diffraction (2θ [°]). The upper chart shows a powder X-ray diffraction spectrum before storage and the bottom one shows that after storage.

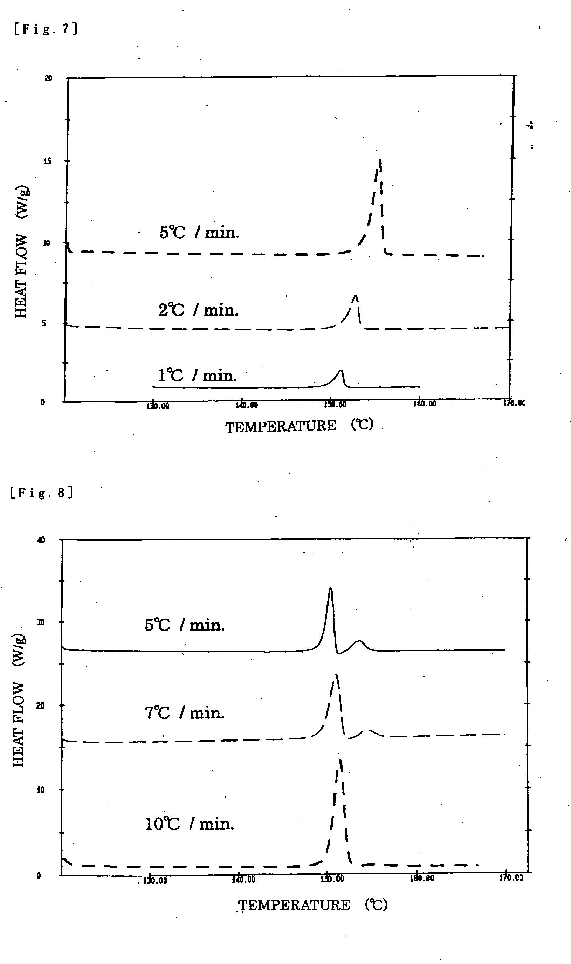

Fig. 7 shows the result of measurement by differential scanning calorimetry in Form I crystal of Compound A. The vertical axis indicates the heat flow and the horizontal axis indicates the temperature. The graphs respectively show the changes at a temperature-raising rate of 5°C, 2°C or 1°C/minute in order from the top.

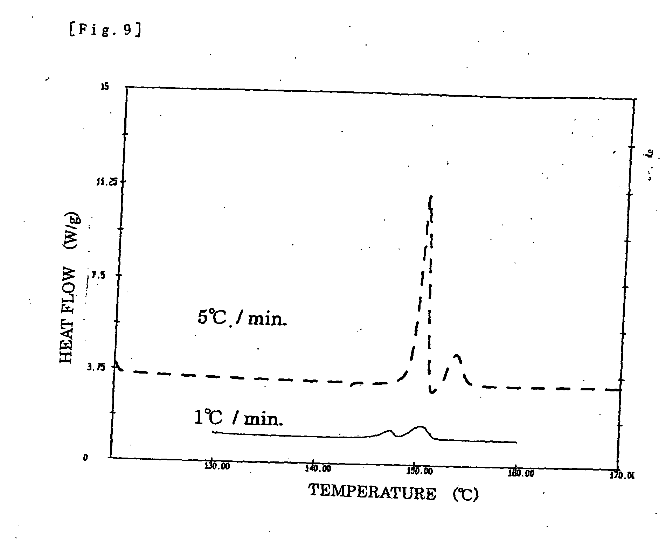

Fig. 8 shows the result of measurement by differential scanning calorimetry in Form II crystal of Compound A. The vertical axis indicates the heat flow and the horizontal axis indicates the temperature. The graphs respectively show the changes at a temperature-raising rate of 5°C, 7°C or 10°C/minute in order from the top.
Fig. 9 shows the result of measurement by differential scanning calorimetry in Form II crystal of Compound A. The vertical axis indicates the heat flow and the horizontal axis indicates the temperature. The graphs respectively show the changes at a temperature-raising rate of 5°C or 1°C/minute in order from the top.

### Best Mode for Carrying Out the Invention

In a powder X-ray diffraction spectrum, Form I crystal of Compound A is characterized in that it shows peaks at the angles of diffraction of 4.9°, 13.7°, 15.5°, 18.6°, 19.7°, and 21.0°. Among these peaks, those at 4.9°, 13.7° and 19.7° are particularly characteristic.

Form I crystal of Compound A, as shown in Experimental section described below, is an important intermediate for preparation of Form II crystal which is useful as drug bulk powder.

In a powder X-ray diffraction spectrum, Form II crystal of Compound A is characterized in that it shows peaks at the angles of diffraction of 12.4°, 13.3°, 17.1°, 20.8°, 22.2°, and 25.9°. Among these peaks, those at 12.4°, 17.1° and 20.8° are particularly characteristic.

Form II crystal of Compound A, as shown in Experimental section described below, is very stable in a condition of high humidity and also stable in a solvent for recrystallization, allowing the synthesis in large quantities on an industrial scale.

### Preparation of Form I crystal

Form I crystal can be prepared, for example, as follows.
(1) Step of dissolution
   Compound A is dissolved in an organic solvent under heating. The organic solvent includes preferably aromatic hydrocarbon solvents such as benzene, toluene, and particularly preferred is toluene. The organic solvent may be used, preferably, in the amount of 5 to 30 parts (w/w), more preferably 10 to 20 parts (w/w), and particularly 12 to 15 parts (w/w), for 1 part of Compound A. The heating temperature is variable depending on the kind and the amount of the organic solvent used, and in general it is kept preferably at 60-90°C, and particularly at 70-80°C. In this step, the reaction is preferably carried out in an inert gas such as nitrogen and argon.
   Compound A used in this process may be produced, for example, according to the method as described in Patent Document 1, and preferably used after pre-treatment with active carbon. Compound A can be used in any form of crystals or in an amorphous form.
   In order to remove insoluble materials, the solution may be filtered. In order to prevent deposition of crystals during filtration, it is appropriate to use a filter funnel equipped with a warming apparatus. When deposition of crystals is observed in the filtrate, the crystals should be re-dissolved under heating after filtration.
(2) Step of cooling
   The solution is rapidly cooled to deposit crystals. In this step, it is preferable to use a crystallization apparatus equipped with a warming function for dissolving deposit and a stirrer function for cooling. This step is preferably carried out under an inert gas atmosphere such as nitrogen and argon.
   Preferred cooling temperature is 0 to 20°C. After reaching the cooling temperature, the crystallization is preferably conducted for a period of 0.5 to 5 hours. In addition, it is appropriate to add Form I crystal as a seed. The seed may preferably be added in an amount of 3-5% by weight for Compound A. Preferred cooling rate is 20-40°C/minute.
(3) Collection of crystals and the step of drying

The deposited crystals are collected by a conventional means such as filtration and centrifugation, and dried. The drying may be carried out under reduced pressure or with a drying agent, and in particular preferably under reduced pressure, for example, at a temperature of 50-70°C under pressure below 10 mmHg for a period of 6-24 hours.

### Preparation of Form II crystal

Form II crystal can be prepared, for example, as follows.
(1) Step of dissolution
   Compound A is dissolved in an organic solvent under heating. The organic solvent includes preferably an alcohol solvent such as methanol, ethanol, and 2-propanol; a carboxylic acid ester solvent such as ethyl acetate and isopropyl acetate; a nitrile solvent such as acetonitrile; and an aromatic hydrocarbon solvent such as toluene; and particularly preferred one is isopropyl acetate. Other operation may be conduced in the same manner as in Form I crystal.
(2) Step of cooling
   The solution is slowly cooled to deposit crystals. The preferred cooling temperature is 0-20°C, particularly 5-10°C. After reaching the cooling temperature, the crystallization is carried out for a period of 0.5 to 5 hours, particularly 2 to 3 hours. In addition, when the solution is cooled to 60°C, it is appropriate to add Form II crystal as a seed. The seed may preferably be added in an amount of 3-5% by weight for Compound A. Preferred cooling rate is 0.5-5°C/minute when the seed is added, and 0.5-1°C/minute when the seed is absent. Other operation may be conduced in the same manner as in Form I crystal.
(3) Collection of crystals and the step of drying

The operation may be conduced in the same manner as in Form I crystal.

### Industrial preparation of Form II crystal

Highly pure Form II crystal which is useful as drug bulk powder can be prepared according to the following process for industrial preparation.

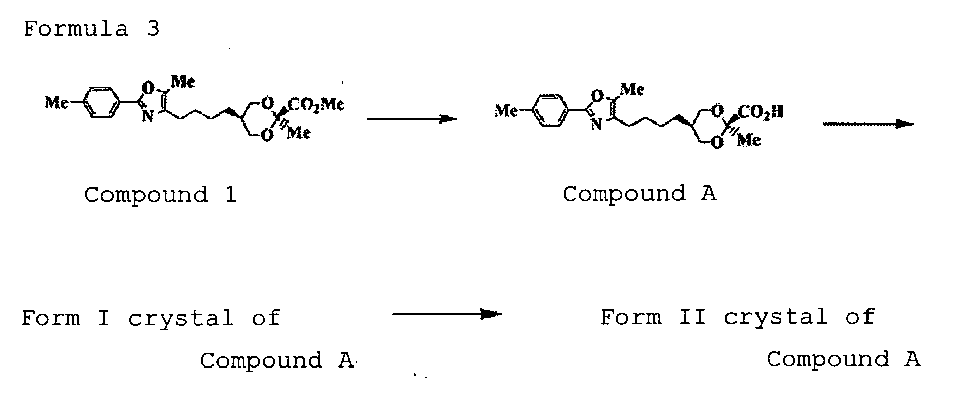

Compound 1 can be produced in the same manner as the process described in Patent Document 1. The resulting Compound 1 may be hydrolyzed directly without purification by column chromatography (for example, see Patent Document 1) to give Compound A. The resulting Compound A is formed (crystallized) into Form I crystal, which is then recrystallized to give highly pure Form II crystal of Compound A. Form I crystal and Form II crystal may be prepared as mentioned above.

In comparison with the prior art process, no purification step by column chromatography is required in the process of the invention; thus, the process of the invention is very useful as a synthetic process for mass production on an industrial scale. Form I crystal of Compound A is an important intermediate in preparation of Form II crystal of Compound A.

### Examples

The invention will be illustrated in more detail by the following Reference Examples, Examples, and Experimental

### Examples.

The powder X-ray diffraction spectra were measured by means of a Rigaku Corporation's RAD-2B (target: Cu; Voltage:40kV; current: 20mA; scanning speed: 4°/minute; measurement error: 2θ ± 0.2°).

The differential scanning calorimetry in Example 1 and Example 2 was made by means of a Perkin Elmer DSC7 (temperature-raising rate: 10°C/minute; the range of measurement: 50°C (constant temperature 1 minute) → 180°C). The differential scanning calorimetry in Experimental Example 4 was made by means of Shimadzu Corporation's DSC-50 (temperature-raising rate: 2°C/minute; the range of measurement: room temperature → 220°C).

The differential scanning calorimetry in Experimental Example 5 was made by means of a Perkin Elmer DSC7 (for temperature-raising rate of 1°C/minute, the range of measurement: 130°C → 160°C; and for temperature-raising rate of 2, 5, 7 or 10°C/minute, the range of measurement: 120°C → 170°C) .

The purity of Compound A in Experimental Example 6 was determined as follows.
Apparatus: Agilent 1100 Series (Agilent Co.)
Column: Cadenza CD-C18 250x4.6 mm (Imtact Co.)
Column temperature: 40°C
Mobile phase: acetonitrile/water/methanesulfoic acid = 550/450/1
Flow rate: 0.7 mL/minute
Detector: UV detector
Wavelength of measurement: 240 nm

### Reference Example 1: Production of Compound A (1)

According to the method described in Patent Document 1, Compound 1 (a methyl ester derivative of Compound A) was synthesized. This compound 1 (250 mg) was then dissolved in 2.5 ml of methanol, to which was then added a solution of 51 mg of sodium hydroxide in 0.6 ml of water, and the mixture was refluxed for 1 hour. After cooling, the reaction mixture was poured into ice water, acidified with diluted hydrochloric acid, and extracted with ethyl acetate. The organic layer was washed with saturated brine, dried on anhydrous magnesium sulfate, and concentrated. Diethyl ether was added to the residue, and the resulting deposit was collected by filtration to give 231 mg of Compound A.
¹H-NMR (δ: CDCl₃) : 1.00-1.12 (2H, m), 1.26-1.46 (2H, m), 1.57-1,77 (2H, m), 1.57 (3H, s), 2.04 (1H, m), 2.31 (3H, s), 2.37 (3H, s), 2.52 (2H, t), 3.50 (2H, t), 3.98 (2H, dd), 7.23 (2H, d), 7.85 (2H, d), 9.38 (1H, br).

### Reference Example 2: Production of Compound A (2)

### Step 1: Production of c-5-[5-acetyl-5-(p-toluoylamino)pentyl]-2-methyl-1,3-dioxa ne-r-2-carboxylic acid

In 192 mL of N,N-dimethylformamide was dissolved 64.0 g of methyl c-5-(4-iodobutyl)-2-methyl-1,3-dioxane-r-2-carboxylate synthesized according to the method described in Patent Document 1 and 34.4 g of 1-(p-toluoylamino)-2-propanone, and 13. 6 g of 85% potassium hydroxide was added thereto and stirred at room temperature for 3 hours. Thereafter, 385 mL of water was added to the reaction mixture, and extracted with 385 mL of toluene. The extracted organic layer was successively washed with 137 mL of 1% aqueous hydrochloric acid solution and 128 mL of water, and dried on anhydrous magnesium sulfate to give a toluene solution of the objective compound.

### Step 2: Production of Compound 1

To a toluene solution of c-5-[5-acetyl-5-(p-toluoylamino)pentyl]-2-methyl-1,3-dioxa ne-r-2-carboxylic acid obtained in Step 1, was added 28.6 g of oxalyl chloride, and the mixture was allowed to react at 50°C for 3 hours. After the reaction completion, 11.8 g of water and 210 g of 15% aqueous potassium hydroxide solution were successively added to wash the organic layer; the organic layer was further washed with 76 mL of water to give a toluene solution of crude Compound 1.

### Step 3: Production of Compound A

To a toluene solution of Compound 1 prepared in Step 2 was added an aqueous sodium hydroxide solution (prepared from 15.0 g of sodium hydroxide and 72.6 g of water) and 36 mL of methanol, and the mixture was stirred at 50°C. After the reaction completion, the mixture was neutralized with conc. hydrochloric acid, and extracted with 363 mL of ethyl acetate, and the organic layer was washed with 145 mL of water. The organic layer was concentrated under reduced pressure, and then 145 mL of toluene was added and concentrated again to give crude Compound A.

### Example 1: Preparation of Form I crystal of Compound A

Compound A (0.5 g) prepared in Reference Example 1 was dissolved in toluene (6 ml) with stirring under heating on an oil bath at 90°C. The solution mixture, which was confirmed visually, was allowed to stand for 5 minutes and then filtered on heating under pressure. The filtrate after filtration was immediately ice-cooled on an ice bath with stirring and the stirring continued for 10 minutes. The deposited crystals were collected by filtration, washed with toluene (1ml), and dried at 60°C under reduced pressure (5 mmHg) to give the objective compound (460 mg).
mp. 154-157°C

Fig. 1 shows a chart of powder X-ray diffraction spectrum of the resulting crystal. It is apparent that Form I crystal shows peaks at the angles of diffraction of 4.9°, 13.7°, 15.5°, 18.6°, 19.7°, and 21.0°. Among these peaks, those at 4.9°, 13.7° and 19.7° are particularly characteristic.

The result of measurement of this crystal by differential scanning calorimetry indicated that the endotherm began at 154-156°C and reached the peak at 159-160°C.

### Example 2: Preparation of Form II crystal of Compound A (1)

To Compound A (1.0 g) prepared according to the method of Reference Example 1 was added toluene (12 ml), and the mixture was dissolved under heating at 90°C with stirring on an oil bath. The solution mixture, which was confirmed visually, was allowed to stand for 5 minutes and then filtered on heating under pressure. The filtrate after filtration was allowed to slowly cool to 25°C and stand for 2 hours. The deposited crystals were collected by filtration, washed with toluene (2 ml), and dried at 60°C under reduced pressure (5 mmHg) to give the objective compound (890 mg).
mp. 150-153°C

Fig. 2 shows a chart of powder X-ray diffraction spectrum of the resulting crystal. It is apparent that Form II crystal shows peaks at the angles of diffraction of 12.4°, 13.3°, 17.1°, 20.8°, 22.2°, and 25.9°. Among these peaks, those at 12.4°, 17.1° and 20.8° are particularly characteristic.

The result of measurement of this crystal by differential scanning calorimetry indicated that the endotherm began at 149-150°C and reached the peak at 153-154°C.

### Example 3: Preparation of Form II crystal of Compound A (2)

To the crude product of Compound A (10.0 g) prepared according to the process of Reference Example 1 was added isopropyl acetate (150 ml), and the mixture was dissolved under heating at an inner temperature of 80-90°C with stirring. The solution mixture, which was confirmed visually, was allowed to stand for 30 minutes and then filtered on heating under pressure. After filtration, the filtrate was cooled to 10°C at a rate of 0.5°C/minute and allowed to stand for 0.5 hour. The deposited crystals were collected by filtration, washed with isopropyl acetate (5 ml), and dried at 60°C under reduced pressure (5 mmHg) to give the objective compound (8.5 g).
mp. 150-153°C

The powder X-ray diffraction spectrum was identical with that of Example 2.

### Example 4: Preparation of Form II crystal of Compound A (3)

To the crude product of Compound A prepared in Reference Example 2 was added 145 ml of toluene and the mixture was dissolved under heating with stirring, to which was added 0.7 g of Form I seed crystals under cooling with stirring, and the mixture was cooled to room temperature. The resulting suspension was filtered, and the crystals were dried at 50°C under reduced pressure to give 24.2 g of Form I crystals of Compound A. Thus resulting Form I crystals were dissolved in 360 mL of isopropyl acetate under heating at 80°C, and the solution was allowed to cool to room temperature at a rate of 0.5-1.0°C/minute with stirring. The resulting suspension was filtered. The crystals were dried at 50°C under reduced pressure to give 19.8 g of Form II crystals of Compound A. Experimental Example 1: Investigation of hygroscopicity

Form I crystals and Form II crystals of Compound A, approximately 1 g each, were precisely weighed in weighing bottles, which were then kept in a desiccator of 93% RH at 25°C. After the lapse of 3 days, 7 days and 14 days, the weight change was observed to measure the amount of absorbed moisture. In addition, the powder X-ray diffraction spectra were measured before and after preservation. The results are shown in Fig. 3 (weight change of Form I crystal), Fig. 4 (weight change of Form II crystal), Fig. 5 (powder X-ray diffraction spectrum of Form I crystal), and Fig. 6 (powder X-ray diffraction spectrum of Form II crystal).

In Form I crystals, as shown in Fig. 3, an increase of weight by 2.3% caused by absorption of moisture was observed. Further, as shown in Fig. 5, transformation of crystalline form (from Form I to Form II) was recognized.

On the other hand, in Form II crystal as shown in Fig. 4, no change of weight caused by absorption of moisture was observed. Further, as shown in Fig. 6, no transformation of crystalline form (from Form II to Form I) was recognized, too.

From the above results, it was suggested that Form II crystal is useful as drug bulk powder since it is much more stable than Form I crystal in a highly humid condition and causes no transformation of crystalline form.

### Experimental Example 2: Investigation on the solvents used in recrystallization of Compound A

A certain amount of Compound A was precisely weighed, to which was added a variety of solvents to give a solution under heating (80-90°C). The solution was cooled to deposit crystals, of which the powder X-ray diffraction spectra were measured to determine the crystalline form. Table 1 shows the results.

**Table 1**

| Solute (mg) | Solvent for Recrystallization | Solvent Amt. (w/w) | Crystalline Form |
|---|---|---|---|
| 99.7 | Methanol | 5 | Form I+Form II |
| 100.1 | Methanol | 10 | Form I+Form II |
| 246.0 | Methanol | 12 | Form II |
| 99.8 | Ethanol | 10 | Form II |
| 100.4 | Isopropanol | 15 | Form II |
| 503.9 | Isopropanol | 20 | Form II |
| 249.5 | Acetonitrile | 30 | Form II |
| 98.3 | Acetonitrile | 50 | Form I+Form II |
| 99.9 | Ethyl acetate | 10 | Form I+Form II |
| 249.5 | Ethyl acetate | 15 | Form II |
| 99.5 | Ethyl acetate | 20 | Form II |
| 200.4 | Toluene | 15 | Form I |
| 200.9 | Toluene | 15 | Form II |
| 10 g | Isopropyl acetate | 15 | Form II |

As shown in Table 1, Form II crystal can be recrystallized from a variety of solvents including toluene and easily prepared. Contrarily, Form I crystal can be prepared only by recrystallization from toluene as solvent.

Recrystallization from toluene was further investigated since the recrystallization from toluene affords not only Form I crystal but also Form II crystal. The results are shown in Experimental Example 3 and Experimental Example 4 as mentioned below.

### Experimental Example 3: Investigation of cooling step

In case of recrystallization of Compound A from toluene, the influence of cooling step on the crystalline form was investigated.

A certain amount of Compound A was precisely weighed, to which was added a certain amount of toluene, and the mixture was dissolved under heating (80-90°C) . The solution was cooled to room temperature at a variety of rate to deposit crystals, of which the powder X-ray diffraction spectra were measured to determine the form of deposited crystals. Table 2 indicates the results.

**Table 2**

| Cooling rate | Crystalline form |
|---|---|
| 0.5-1.0°C/minute | Form II |
| 1.0-2.0°C/minute | Form II |
| 2.0-5.0°C/minute | Form II |
| 20-40°C/minute | Form I |

As shown in Table 2, it is necessary to rapidly cool the solution (20-40°C/minute) for crystallization in order to prepare Form I crystal. Contrarily, the solution may slowly be cooled (0.5-5°C/minute) for the preparation of Form II crystal. Thus, the synthesis of a large amount of Form II crystal is allowed.

### Experimental Example 4: Investigation of transformation mediated by solvents

The influence of toluene (solvent) on the crystalline form of Compound A was investigated.

Form I crystals and Form II crystals of Compound A, 50 mg each, were weighed and respectively suspended in 1 ml of toluene, and stirred overnight. Thereafter, the crystals were separated by filtration and dried, of which the crystalline form was confirmed by a differential scanning calorimetry. As a result, it became apparent that no transformation of crystalline form occurs in form II crystal after stirring overnight to maintain the original crystalline form before stirring, but Form I crystal is transformed into Form II crystal.

From the above results, it was suggested that Form II crystal is useful as drug bulk powder since it is much more stable than Form I crystal in solvents for recrystallization.

### Experimental Example 5: Investigation of thermodynamic stability

The thermodynamic stability of the crystal of Compound A was investigated by differential scanning calorimetry.

The differential scanning calorimetry was carried out at a temperature-raising rate of 1, 2 or 5°C/minute for Form I crystal of Compound A and at 1, 5, 7 or 10°C/minute for Form II crystal. The results are shown in Fig. 7 (a chart of differential scanning calorimetry in Form I crystal), Fig. 8 and Fig. 9 (a chart of differential scanning calorimetry in Form II crystal).

As shown in Fig. 7, Form I crystal showed only one endothermic peak regardless of the temperature-raising rate. On the other hand, as shown in Fig. 8 and Fig. 9, no peak of Form I crystal was confirmed in Form II crystal at a temperature-raising rate of 10°C/minute, but a peak resulting from Form I crystal was recognized as the temperature-raising rate was lower like 7°C/minute and 5°C/minute, and its area became large. The result is much more remarkable at 1°C/minute .

### Experimental Example 6: Investigation of a process for purification of crude product of Compound A

In the prior art, as mentioned above, a step of purification by column chromatography is necessarily required during the process for producing Compound A. In order to remove such a purification step from an industrial-scale production process, an alternative purification process by crystallization and recrystallization was investigated.

First, crystallization and recrystallization of Form II crystal of Compound A were investigated using a crude product of Compound A for which no purification by column chromatography had been done. Specifically, 5 parts by volume (w/w) of toluene was added to one part of crude product of Compound A; the mixture was dissolved under heating, and then cooled to room temperature with stirring at a rate of 0.5-1.0°C/minute; the suspension was filtered. The resulting crystals were dried at 50°C under reduced pressure to give Form II crystal of Compound A (crystallization). To the resulting Form II crystal of Compound A was added again 15 parts by volume (w/w) of isopropyl acetate; the mixture was dissolved under heating, and then cooled to room temperature with stirring at a rate of 0.5-1.0°C/minute; the suspension was filtered. The resulting crystals were dried at 50°C under reduced pressure to give Form II crystal of Compound A (recrystallization). Table 3 shows the results.

**Table 3**

| Purity of Compound A in crude state | Purity of Compound A after crystallization | Purity of Compound A after recrystalliztion |
|---|---|---|
| 66% | 91.7% | 95.4% |
| 77% | 93.7% | 96.2% |

As shown in Table 3, it was not possible to obtain the desirable drug bulk powder of which the purity is 99% by crystallization and recrystallization of Form II crystal, because of lower effect of removal of impurity.

In this situation, the present inventors considered that it would be possible to enhance the efficiency of purification by preparing Form I crystal at the first stage without forming Form II crystal, and tried such a process. Specifically, the crude product of Compound A was dissolved in 5 parts by volume (w/w) of toluene under heating, to which was added a seed crystal, and the mixture was rapidly cooled under ice-cooling with stirring and then the suspension was filtered. The resulting crystals were dried at 50°C under reduced pressure to give Form I crystal of Compound A (crystallization). To the resulting Form I crystals of Compound A was added again 15 parts by volume (w/w) of isopropyl acetate, and the mixture was dissolved under heating, and then cooled to room temperature at a rate of 0.5-1.0°C/minute with stirring, and the suspension was filtered. The crystals were dried at 50°C under reduced pressure to give Form II crystals of Compound A (recrystallization). The results are shown in Table 4.

**Table 4**

| Purity of Compound A in crude state | Purity of Compound A after crystallization | Purity of Compound A after recrystalliztion |
|---|---|---|
| 64% | 98.4% | 99.2% |
| 66% | 97.7% | 99.3% |
| 70% | 98.5% | 99.4% |

As shown in Table 4, it was found that the efficiency of purification was greatly improved by crystallization of Form I crystals at the first stage. Recrystallization of the resulting crystals gave Form II crystals. Thus, Form II crystals could be prepared in 99% or higher purity.

From these results, it was possible to establish a process for preparing Form II crystal of Compound A in 99% or higher purity from crude products of Compound A without purification using column chromatography which is unsuitable for a large scale synthesis. Therefore, it is apparent that Form I crystal of Compound A is an important intermediate for Form II crystal which is useful as drug bulk powder.

### Industrial Applicability

Form II crystal of Compound A is stable in a highly humid condition and even in a solvent for recrystallization and can easily be prepared on an industrial scale.

On the other hand, Form I crystal is an important intermediate for preparation of Form II crystal which is useful as drug bulk powder.

## Claims

1. A crystal of 2-methyl-c-5-[4-[5-methyl-2-(4-methylphenyl)-1,3-oxazol-4-yl]butyl]-1,3-dioxane-r-2-carboxylic acid, which has peaks at the angles of diffraction of at least 4.9°, 13.7°, and 19.7° in a powder X ray diffraction spectrum.

2. The crystal as claimed in Claim 1, further having peaks at the angles of diffraction of 15 5°, 18.6° and 21.0°.

3. A crystal of 2-methyl-c-5-[4-[5-methyl-2-(4-methylphenyl)-1,3-oxazol-4-yl]butyl]-1,3-dioxane-r-2-carboxylic acid, which has peaks at the angles of diffraction of at least 12.4°, 17.1°, and 20.8° in a powder X-ray diffraction spectrum.

4. The crystal as claimed in Claim 3, further having peaks at the angles of diffraction of 13,3°, 22.2° and 25.9°.

5. A pharmaceutical composition comprising as an active ingredient the crystal as claimed in Claim 1 or 2.

6. A pharmaceutical composition comprising as an active ingredient the crystal as claimed in Claim 3 or 4.

7. A preventive or therapeutic agent comprising as an active ingredient the crystal as claimed in Claim 1 or 2 for use in coronary artery diseases, cerebral infarction, hyperlipemia, arteriosclerosis, diabetes mellitus, hypertension, or obesity.

8. A preventive or therapeutic agent comprising as an active ingredient the crystal as claimed in Claim 3 or 4 for use in coronary artery diseases, cerebral infarction, hyperlipemia, arteriosclerosis, diabetes mellitus, hypertension, or obesity.

9. A process for preparing the crystal as claimed in Claim 3 or 4, which process comprises the steps of:
(i) preparing the crystal as claimed in Claim 1 or 2 from a crude product of 2-methyl-c-5-[4-[5-methyl-2-(4-methylphenyl)-1,3-oxazol-4-yl]butyl]-1,3-dioxane-r-2-carboxylic acid; and
(ii) preparing the crystal as claimed in Claim 3 or 4 from the crystal obtained in the above step (i).
